# EUROPÄISCHE PATENTSCHRIFT

(11) **EP 2 258 264 B1**
(45) Veröffentlichungstag und Bekanntmachung des Hinweises auf die Patenterteilung: **21.12.2011**
(21) Anmeldenummer: 09161829.8
(22) Anmeldetag: 03.06.2009
(51) Int. Cl.: A61B 5/103, A61B 19/00

(54) **Expressregistrierung von Körperregionen**
Express registration of body regions
Enregistrement express de régions du corps

(43) Veröffentlichungstag der Anmeldung: 08.12.2010
(73) Patentinhaber: BrainLAB AG, 85622 Feldkirchen (DE)
(72) Erfinder: Neubauer, Timo, 85586, Poing (DE); Uhing, Frank, 80799, München (DE)
(74) Vertreter: Schwabe - Sandmair - Marx

(56) Entgegenhaltungen:
- EP-A- 2 044 884
- WO-A-2006/106335
- WO-A-2007/117695
- DE-A1-102006 020 399
- US-A1- 2008 228 188

## Beschreibung

Die vorliegende Erfindung bezieht sich auf ein Verfahren zum Bestimmen der Körperregion, insbesondere einer rechten oder linken Seite des Körpers, in der bzw. auf der sich ein anatomischer Körperteil, beispielsweise ein Unterschenkelknochen (insbesondere eine Tibia und/oder Fibula) befindet, der insbesondere im wesentlichen symmetrisch zu einem auf der anderen Seite bzw. in einer anderen Region des Körpers liegenden Körperteil ausgebildet ist.

In chirurgischen Navigationsverfahren (engl.: image guided surgery - IGS) besteht regelmäßig für den Operateur ein Bedarf, Körperteile, die beispielsweise symmetrisch an unterschiedlichen Seiten des Körpers angeordnet sind, der entsprechenden Seite zuzuordnen. Hierbei besteht ein Problem darin, dass z.B. ein rechter und ein linker Unterschenkelknochen abhängig vom Beobachterstandort bzw. der Perspektive, unter welcher der Knochen betrachtet wird, eine deckungsgleiche Form bzw. Ansicht aufweisen können. Dadurch ergibt sich ein Problem bei der Zuordnung des entsprechenden Körperteils des Patienten zu den entsprechenden Körperteilen im vor der Operation hinterlegten Modelldatensatz des Patientenkörpers. Insbesondere ist das Patientenkörperteil nicht mehr eindeutig in dem Modelldatensatz einzuordnen.

Unter einem Patientenkörperteil wird in diesem Zusammenhang der reale Körperteil des Patienten verstanden. Zu unterscheiden ist der Patientenkörperteil von einem virtuellen, von einem Modelldatensatz des realen Patientenkörperteils umfassten Modell des Körperteils. Ein Patientenkörperteil wird hierin auch als anatomischer Körperteil bezeichnet. Bislang wurde die Zuordnung des Patientenkörperteils zu einer bestimmten Körperregion, z.B. die Zuordnung eines Schienbeinknochens (Tibia) zum rechten oder linken Bein des Patienten, durch händische Eingabe des Operateurs in eine Datenverarbeitungsvorrichtung vorgenommen. Dabei können sich jedoch Fehler ergeben, die beispielsweise auf einer Fehleingabe des Benutzers beruhen können. Außerdem kostet die Eingabe Zeit.

DE 10 2006 020 399 A1 offenbart eine Vorrichtung und ein Verfahren zur Verarbeitung diagnostischer Bilddaten, bei dem zuvor gespeicherte Bezeichnungen von Lymphknoten einer mittels eines bildgebenden Diagnosegeräts der detektierten anatomischen Struktur zugeordnet werden. Dabei wird mit einem gespeicherten Referenzdatensatz gearbeitet, der geometrische Informationen über die relevanten anatomischen Strukturen und die Bezeichnungen verschiedener Lymphknoten umfasst.

US 2008/228188 A1 offenbart ein System, das aus einer Datenbank ein generisches Modell eines Körperteils heraussucht, das der Form und Größe eines tatsächlichen Patientenkörperteils am nächsten kommt.

Aufgabe der folgenden Erfindung ist es somit, ein Verfahren zur automatischen Bestimmung der Körperregion, in dem sich der Patientenkörperteil tatsächlich befindet, bereitzustellen, um die Zuordnung des Patientenkörperteils zum Modelldatensatz zu vereinfachen. Insbesondere soll mit dem erfindungsgemäßen Verfahren ermittelt werden, auf welcher Körperseite (d.h. auf der linken oder rechten Hälfte) der Patientenkörperteil angeordnet ist, d.h. ob es sich um einen rechten oder linken Körperteil handelt. Dies wird im Folgenden auch als Seitenbestimmung bezeichnet. Der Begriff der Seitenbestimmung kann allerdings auch alternativ oder zusätzlich die Bestimmung der Körperregion umfassen, in der sich der anatomische Körperteil befindet. Insofern kann der Begriff der Körperregion den Begriff der Körperseite umfassen, insbesondere können die Begriffe austauschbar verwendet werden.

Diese Aufgabe wird gelöst durch den Gegenstand der unabhängigen Ansprüche 1, 12 und 13. Die abhängigen Ansprüche beinhalten vorteilhafte Ausführungsformen der Erfindung. Dabei können die Merkmale unterschiedlicher Ausführungsformen der Erfindung untereinander kombiniert werden.

Vorteilhaft werden zum Bestimmen der Körperregion, in der sich ein anatomischer Körperteil befindet, folgende Schritte durchgeführt: Anatomische Punktdaten, die Informationen über ein räumliches Muster von Lagen von Patienten-Landmarken eines Patienten-Körperteils der dem anatomischen Körperteil entspricht, umfassen, werden bereitgestellt. Bei einer Patienten-Landmarke handelt es sich um eine insbesondere anatomische Landmarke am Körper des Patienten.

Unter einer anatomischen Landmarke wird ein definierter, charakteristischer Punkt einer anatomischen Struktur verstanden, der bei der gleichen anatomischen Struktur mehrerer Patienten stets identisch oder mit hoher Ähnlichkeit wiederkehrt. Typische Landmarken sind beispielsweise die Epikondylen eines Oberschenkelknochens oder die Spitzen der Querfortsätze bzw. des Dornfortsatzes eines Wirbels. Landmarken, die sich am Patienten-Körperteil befinden, werden im Folgenden als Patienten-Landmarken bezeichnet; Landmarken, die in einem Modell des Patienten-Körperteils definiert sind, werden als Modell-Landmarken bezeichnet. Wenn ein Verfahrensschritt im Rahmen dieser Erfindung sowohl auf Patienten-Landmarken als auch auf Modell-Landmarken anwendbar ist oder angewendet wird, so ist im Folgenden lediglich von Landmarken die Rede.

Zu unterscheiden sind anatomische von künstlichen Landmarken. Künstliche Landmarken können an anatomischen Strukturen definiert werden, die üblicherweise nicht bei der gleichen anatomischen Struktur mehrerer Patienten stets identische oder mit hoher Ähnlichkeit wiederkehren. Insbesondere können künstliche Landmarken durch Anbringen einer Markervorrichtung bzw. eines Referenzstern an der anatomischen Struktur und/oder die relative Lage der Markervorrichtung bzw. des Referenzstern bezüglich der anatomischen Struktur definiert werden.

Der Patienten-Körperteil ist insbesondere eine Tibia und/oder Fibula, die dem rechten oder linken Bein des Patienten zugeordnet werden soll, so dass ein chirurgisches Navigationssystem ohne dazu notwendige Eingabe des Benutzers automatisch erkennt, ob sich es beispielsweise um eine rechte oder linke Tibia und/oder rechte oder linke Fibula handelt. Das erfmdungsgemäße Verfahren kann aber auch auf andere Körperteile, beispielsweise eine Scapula oder einen Humerus angewandt werden.

Die anatomischen Punktdaten werden vorteilhaft mit einem Pointer referenziert. Ein Pointer ist ein Stab mit einem oder mehreren, vorteilhaft zwei daran befestigten Markern, wobei der Pointer dazu verwendet werden kann, im Rahmen der Referenzierung einzelne Koordinaten, insbesondere Raumkoordinaten (also dreidimensionale Koordinaten) an einem Körperteil, abzugreifen. Dabei führt ein Benutzer den Pointer (insbesondere einen Teil des Pointers, der eine definierte - vorteilhaft feste - Lage bezüglich des wenigstens einen an dem Pointer angebrachten Markers innehat beispielsweise die Pointerspitze) so an die den Koordinaten entsprechende Position, dass über eine Erfassung des Markers an dem Pointer durch ein chirurgisches Navigationssystem die Position des Pointers bestimmt werden kann. Insbesondere ist die relative Lage zwischen den Markern des Pointers und dem zum Abgreifen von Koordinaten verwendeten Teil des Pointers (insbesondere der Pointerspitze) bekannt. Das chirurgische Navigationssystem ermöglicht dann die Zuordnung der Position (der dreidimensionalen Koordinaten) zu einer vorbestimmten Körperstruktur (insbesondere einem Modelldatensatz der Körperstruktur), wobei die Zuordnung automatisch und/oder durch Eingreifen des Benutzers erfolgen kann. Die Referenzierung erlaubt also, die anatomischen Punktdaten zu digitalisieren und ihre Verarbeitung durch eine digitale Datenverarbeitungseinrichtung zu ermöglichen.

Unter der Lage des Objekts wird eine Beschreibung seiner Position im (insbesondere dreidimensionalen) Raum (die beispielsweise mit kartesischen und/oder sphärischen Koordinaten durch Vektoren beschrieben werden kann) sowie vorteilhaft einer Ausrichtung des Objekts verstanden. Die Ausrichtung ergibt sich z.B. aus einer Richtungsinformation, insbesondere der Richtung eines Vektors von dem Objekt zu einem anderen Objekt. Umfasst die Lage eine Richtungsinformation, so wird dies vorteilhaft als relative Lage bzw. Lage relativ zu einem Objekt bezeichnet.

Unter der Lage einer Patienten-Landmarke sind deren Koordinaten in einem lokalen (beispielsweise auf den Patientenkörper bezogenen) oder globalen (beispielsweise auf eine Arbeitsumgebung wie den Operationssaal bezogenen) Koordinatensystem zu verstehen. Insbesondere wird die Lage einer Patienten-Landmarke als bei der Referenzierung von dem Pointer bzw. der Pointerspitze definierter Punkt ermittelt. Die anatomischen Punktdaten, die Informationen über solche Lagen der Patienten-Landmarken umfassen, werden also erfindungsgemäß bereitgestellt, indem sie von einem chirurgischen Navigationsverfahren bei der Referenzierung aufgenommen werden. Die Patienten-Landmarken können alle in einer Ebene liegen, werden jedoch vorteilhaft so referenziert, dass das nicht der Fall ist (d.h. vorteilhaft liegt wenigstens eine Patienten-Landmarke nicht in einer Ebene mit den anderen Patienten-Landmarken). Dadurch kann die Seitenbestimmung vereinfacht werden.

Ferner werden Landmarken-Daten bereitgestellt, die Informationen über ein räumliches Muster von Lagen von Modell-Landmarken eines Modells des anatomischen Körperteils des Patienten umfassen.

Vorteilhaft sind Informationen über die Lage der Patienten-Landmarken und/oder der Modell-Landmarken relativ zu der Lage wenigstens eines Referenzpunkts auf dem anatomischen Körperteil bzw. im Modell bekannt. Der Referenzpunkt kann beispielsweise durch eine Markervorrichtung gekennzeichnet werden. Insbesondere kann der Referenzpunkt an dem Ort liegen, an dem eine Markervorrichtung an den Patientenkörperteil angrenzt bzw. an dem sie eine Knochenoberfläche durchdringt. Der Referenzpunkt kann alternativ oder zusätzlich in einem Bestandteil einer Markervorrichtung liegen. Eine Mehrzahl von Referenzpunkten, insbesondere zwei oder drei Referenzpunkte, kann die Genauigkeit des erfindungsgemäßen Verfahrens erhöhen.

Der Referenzpunkt im Patientenkörperteil und der Referenzpunkt im Modell sind vorteilhaft einander äquivalent, d.h. sie haben insbesondere eine gleiche oder ähnliche relative Lage bzw. geometrische Beziehung zu bestimmten anderen Körperteilen, insbesondere dem anatomischen Körperteil, der im erfindungsgemäßen Verfahren betrachtet wird.

Eine Markervorrichtung kann ein Referenzstern, ein Pointer und/oder ein einzelner oder mehrere Marker sein.

Aufgabe eines Markers ist, von einer Markererfassungseinrichtung (z.B. einer Kamera oder einem Ultraschallempfänger) erfasst zu werden, so dass seine Lage (d.h. Position und/oder Ausrichtung) im Raum ermittelt werden kann. Solche Marker können aktive Marker sein. Ein aktiver Marker emittiert beispielsweise elektromagnetische Strahlung bzw. Wellen, wobei diese Strahlung im infraroten, sichtbaren und/oder ultravioletten Spektralbereich liegen kann. Der Marker kann aber auch passiv sein, d.h. beispielsweise elektromagnetische Strahlung aus dem infraroten, sichtbaren und/oder ultravioletten Spektralbereich reflektieren. Dazu kann der Marker mit einer Oberfläche versehen sein, die entsprechende Reflektionseigenschaften besitzt. Es ist auch möglich, dass ein Marker elektromagnetische Strahlung bzw. Wellen reflektiert und/oder emittiert, die im Bereich der Radiofrequenzen oder bei Ultraschallwellenlängen liegt bzw. liegen. Ein Marker besitzt vorzugsweise sphärische Form bzw. eine kugelähnliche Gestalt und kann daher als Markerkugel bezeichnet werden, Marker können aber auch eine eckige, beispielsweise würfelige Gestalt aufweisen.

Ein Referenzstern bezeichnet eine Einrichtung, an der mehrere, vorteilhaft drei Marker angebracht sind. Die Marker sind dabei ortsfest und vorteilhaft lösbar an dem Referenzstern angebracht, so dass eine bekannte (vorteilhaft feste) relative Lage der Marker zueinander entsteht. Die relative Lage der Marker zueinander kann für jeden im Rahmen eines chirurgischen Navigationsverfahrens verwendeten Referenzstern individuell unterschiedlich sein, um anhand der relativen Lage der Marker zueinander eine Identifikation des dazugehörigen Referenzstems durch ein chirurgisches Navigationssystem zu ermöglichen. Damit können dann auch die Gegenstände (z. B. Instrumente und/oder Körperteile) identifiziert bzw. voneinander unterschieden werden, an welche der Referenzstern angebracht ist. Der Referenzstern dient dazu, in einem chirurgischen Navigationsverfahren eine Mehrzahl von Markern an einem Gegenstand (beispielsweise einem Knochen bzw. anatomischen Körperteil oder einem medizinischen Instrument) anzubringen, um die Lage des Gegenstandes im Raum (d.h. seine Position und/oder Ausrichtung) erfassen zu können. Ein solcher Referenzstern umfasst insbesondere eine Anbringmöglichkeit an dem Gegenstand (beispielsweise eine Schelle und/oder ein Gewinde) und/oder ein Halteelement, das für einen Abstand der Marker von dem Gegenstand sorgt (um insbesondere die Sichtbarkeit der Marker für eine Markererfassungseinrichtung zu unterstützen) und/oder mit dem Halteelement mechanisch verbundene Markerhalter, an die die Marker angebracht werden können. Wo es aus dem Zusammenhang klar wird, kann der Begriff Referenzstern auch einen Referenzstern mit wenigstens einem daran angebrachten Marker bezeichnen. Ein solches System aus einem Referenzstern und wenigstens einem Marker kann auch Markerstern genannt werden.

Unter einem chirurgischen Navigationssystem bzw. Navigationssystem wird ein System verstanden, das aus wenigstens einer Markervorrichtung, einem Sender, der elektromagnetische Wellen bzw. Strahlung und/oder Ultraschallwellen emittiert, und einem Empfänger, der elektromagnetische Wellen bzw. Strahlung und/oder Ultraschallwellen empfängt, sowie einer elektronischen Datenverarbeitungsvorrichtung, die an den Empfänger und/oder den Sender angeschlossen ist, besteht. Die Datenverarbeitungsvorrichtung (z. B. ein Computer) umfasst dabei einen Prozessor (CPU), einen Arbeitsspeicher, vorteilhaft eine Anzeigemöglichkeit (z. B. eine visuelle Anzeigemöglichkeit wie einen Monitor und/oder eine Audioanzeigemöglichkeit wie einen Lautsprecher) sowie vorteilhaft einen permanenten Datenspeicher. Die Datenverarbeitungsvorrichtung verarbeitet dabei Navigationsdaten, die von dem Empfänger an sie weitergegeben werden und kann vorteilhaft Hinweisinformationen über die Anzeigemöglichkeit an einen Anwender ausgeben. Die Navigationsdaten können in dem permanenten Datenspeicher abgelegt werden und beispielsweise mit Daten verglichen werden, die dort zuvor bereitgestellt worden sind.

Werden hierin Daten "bereitgestellt", so bedeutet dies, dass sie für die Nutzung durch das erfindungsgemäße Verfahren bereit sind. Diesen Zustand des "Bereitstehens" können die Daten z.B. durch Erfassen der Daten (z.B. durch Analysegeräte) oder durch Eingeben der (z.B. über Schnittstellen) erreichen. Die Daten können diesen Zustand auch dadurch haben, dass sie in einem Speicher (z.B. ROM, CD, Festplatte) abgespeichert und so für eine Verwendung im Rahmen des erfindungsgemäßen Verfahrens bereit stehen.

Die bereitgestellten Daten von wenigstens Teilen des Patientenkörpers (insbesondere des im erfindungsgemäßen Verfahren betrachteten Patienten-Körperteils) können beispielsweise durch bildgebende medizintechnische Verfahren gewonnen werden. Darunter versteht man vorteilhaft apparative Verfahren der Radiologie, wie etwa Computertomographie (CT), Röntgentomographie, Magnetresonanztomographie (MRT bzw. MRI), herkömmliches Röntgen, Sonographie bzw. Ultraschalluntersuchungen, Positronen-Emissions-Tomographie.

Die Landmarken-Daten beinhalten insbesondere Informationen über die körperseitenabhängigen geometrischen Unterschiede zwischen den Modell-Landmarken. Insbesondere sind dies körperseitenabhängige Unterschiede in der relativen Lage zwischen einer oder mehreren Modell-Landmarken und der Lage des Referenzpunktes, es können aber auch körperabhängige Unterschiede über die Lage nur der Modell-Landmarken zueinander sein. Die Landmarken-Daten können auch Informationen über die relative Lage einer Modell-Landmarke zu einer anderen Modell-Landmarke beinhalten, womit beispielsweise Informationen über die relativen Abstände und/oder ein geometrisches Muster, das sich aus der räumlichen Anordnung der Modell-Landmarken und insbesondere des Referenzpunktes ergibt, gemeint ist. Vorteilhaft beinhalten die Landmarken-Daten Informationen über die Unterschiede solcher Abstände und/oder relativer Lagen und/oder Muster der Modell-Landmarken und insbesondere des Referenzpunktes bezüglich der Anordnung einander entsprechender Modell-Landmarken in unterschiedlichen Körperregionen, so z.B. über die Unterschiede zwischen der Anordnung der Modell-Landmarken und insbesondere des Referenzpunktes auf einer rechten Tibia und Fibula und der Anordnung der entsprechenden Modell-Landmarken und insbesondere des Referenzpunktes auf einer linken Tibia und Fibula.

Der Referenzpunkt an dem Patienten-Körperteil ist vorteilhaft Bestandteil des Satzes von Patienten-Landmarken; schließlich stellen sowohl die Landmarken als auch der Referenzpunkt insbesondere stereotaktisch definierte bzw. referenzierte Punkte dar und können somit in einen gemeinsamen Datensatz aufgenommen werden. Die anatomischen Punktdaten umfassen dann vorteilhaft Informationen über die Lage des Referenzpunkts am Patienten-Körperteil, die Landmarken-Daten umfassen dann vorteilhaft auch Informationen über die Lage des Referenzpunkts im Modell des anatomischen Körperteils. Dann ist der Referenzpunkt im Modell des anatomischen Körperteils vorteilhaft Bestandteil des Satzes von Modell-Landmarken. Der Referenzpunkt stellt dann sowohl im Satz von Patienten-Landmarken als auch im Satz von Modell-Landmarken eine künstliche Landmarke dar, wie in Anspruch 1 definiert.

Die Lage des Referenzpunkts ist vorteilhaft dann bekannt bzw. definiert, wenn ein Winkel α zwischen zwei Geraden bzw. Ebenen, von denen z.B. eine eine Landmarke auf dem Malleolus lateralis beinhaltet und senkrecht zur Tibiaachse steht und die andere z.B. sowohl die Landmarke auf dem Malleolus lateralis als auch eine Landmarke auf dem Malleolus medialis beinhaltet, klein ist (beispielsweise ungefähr gleich 0°, oder 2° oder mit geringem Betrag kleiner als 0°, beispielsweise -1° oder -2° beträgt). Dies kann der Fall sein, wenn die Lage der Landmarken bei der Referenzierung nicht sorgfältig genug festgelegt wird. Wenn der Winkel α jedoch eindeutig bestimmbar ist, also beispielsweise einige Grad (beispielsweise 5° oder 10° oder mehr) beträgt, kann vorteilhaft auf die Festlegung des Referenzpunkts verzichtet werden. Wenn der Referenzpunkt zur erfolgreichen Seitenbestimmung jedoch notwendig ist, ist ein Referenzstern, mit dem seine Position festgelegt wird, vorteilhaft nach anterior oder posterior ausgerichtet (also eindeutig in einen der Halbräume weist, die von der Frontalebene der Tibia definiert werden), insbesondere ist ferner bekannt ob er medial oder lateral beispielsweise an der Tibia angebracht ist. Dies ermöglicht dann eine Bestimmung der Körperregion, in welcher Körperhälfte sich der anatomische Körperteil befindet, insbesondere der rechten oder linken Körperseite. Ebenso kann auf die Festlegung des Referenzpunkts verzichtet werden, wenn wie oben beschrieben, nicht alle Patienten-Landmarken sich in einer Ebene befinden. Eine Seitenbestimmung kann dann erfolgen, indem das charakteristische Muster der relativen Lagen bzw. der Anordnung der Patienten-Landmarken zueinander mit einer aus einem vor Beginn der Operation aufgenommenen Modell-Datensatz bekannten Lage der Modell-Landmarken verglichen wird. Die Seitenbestimmung kann dann unter Berücksichtigung einer ausgezeichneten Lage der wenigstens einen Patienten-Landmarke und Modell-Landmarke erfolgen, wobei die ausgezeichnete Lage eine Lage ist, die sich nicht in einer Ebene mit den Lagen der verbleibenden Patienten-Landmarken bzw. Modell-Landmarken befindet.

Vorteilhaft umfassen die anatomischen Punktdaten und/oder Landmarken-Daten also auch Informationen über ein Muster, insbesondere räumliches Muster, das nicht nur aus den relativen Lagen der Patienten-Landmarken und/oder Modell-Landmarken jeweils zueinander aufgebaut wird, sondern insbesondere zusätzlich aus den relativen Lagen der Patienten-Landmarken und/oder Modell-Landmarken relativ zum Referenzpunkt (am Patienten-Körperteil bzw. im Modell des anatomischen Körperteils) aufgebaut wird. Ein Vergleich der anatomischen Punktdaten und Landmarken-Daten umfasst dann beispielsweise einen Vergleich des räumlichen Musters von Lagen von Patienten-Landmarken, Modell-Landmarken und insbesondere mindestens einem Referenzpunkt, wobei gegebenenfalls die Lage des Referenzpunkts vorteilhaft sowohl am Patientenkörper als auch im Modell berücksichtigt wird. Unter einem räumlichen Muster wird im Rahmen dieser Erfindung insbesondere eine Verteilung von Lagen im Raum, beispielsweise ein definiertes mehrdimensionales (vorteilhaft dreidimensionales oder zweidimensionales) Bild von Koordinaten und/oder von Vektoren im Raum und/oder in der Fläche (insbesondere in ebener Projektion des Raums) verstanden.

Die Landmarken-Daten sind vorteilhaft vorbekannt, d.h. zu Beginn des Verfahrens als gegeben anzusehen. Insbesondere werden die Landmarken-Daten mithilfe eines bildgebenden Verfahrens (dem der betrachtete Patienten-Körperteil unterworfen wird) ermittelt.

Gemäß Anspruch 1 wird angenommen, dass die abgegriffenen Patienten-Landmarken ihre Entsprechung im Satz der Modell-Landmarken haben, also die gleichen Landmarken darstellen. Es wird angenommen, dass die Patienten-Landmarken die gleichen Landmarken darstellen wie die Modell-Landmarken. Hierunter ist zu verstehen, dass zu einer Patienten-Landmarke eine Modell-Landmarke gefunden werden kann, deren Lage im Modell des anatomischen Körperteils vergleichbar bzw. ähnlich zu der Lage der Patienten-Landmarke im realen Patientenkörper ist. Insbesondere haben die Patienten-Landmarken jeweils zueinander relative Lagen, die den relativen Lagen von Modell-Landmarken zueinander entsprechen, so dass auf dieser Grundlage eine eindeutige Zuordnung des Satzes von Patienten-Landmarken zu einem Satz von Modell-Landmarken möglich ist. Vorteilhaft wird also der Satz Modell-Landmarken aus einer Abbildung des anatomischen Patienten-Körperteils gewonnen, stellt also insbesondere keinen allgemeinen Satz von Modell-Landmarken für beispielsweise einen regelrecht geformten anatomischen Körperteil dar, der für eine Mehrzahl von Patienten verwendet wird.

Vorteilhaft hat auch der Referenzpunkt eine Entsprechung sowohl im Patientendatensatz als auch im Modelldatensatz. Insbesondere ist die Lage des Referenzpunkts relativ zu den Patienten-Landmarken vergleichbar zur Lage des Referenzpunkts relativ zu den Modell-Landmarken, d.h. die Lagebeziehungen sind einander vergleichbar bzw. äquivalent. Das heißt, die Lage des Referenzpunkts relativ zu wenigstens einer Patienten-Landmarke findet ihre geometrische Entsprechung in der relativen Lage des Referenzpunkts zu wenigstens einer Modell-Landmarke, die der wenigstens einen Patienten-Landmarke entspricht.

Vorteilhaft werden die Landmarken-Daten mit den anatomischen Punktdaten verglichen, so dass basierend auf den Landmarken-Daten und den anatomischen Punktdaten die Körperregion bestimmt wird, in der sich der Patienten-Körperteil befindet. Insbesondere erfolgt diese Bestimmung auf Grundlage des Ergebnisses aus dem Vergleich der Landmarken-Daten und der anatomischen Punktdaten. Insbesondere werden die Lagen der Landmarken relativ zueinander im Patientendatensatz (d.h. der Patienten-Landmarken) mit den Lagen der Landmarken relativ zueinander im Modelldatensatz (d.h. der Modell-Landmarken) miteinander verglichen. Aus diesem Vergleich kann sich ergeben, dass dem Satz an Patienten-Landmarken ein bestimmter Satz an Modell-Landmarken entspricht, der eine eindeutige Zuordnung des Patienten-Körperteils zu einem Modell des anatomischen Körperteils ermöglicht. So kann beispielsweise ermittelt werden, dass es sich um eine rechte und nicht eine linke Tibia handelt. In diesem Fall bezeichnet die Körperregion, die bestimmt werden soll, die rechte Seite bzw. Hälfte des Patientenkörpers und/oder das rechte Bein des Patienten. Analog soll bei Vorliegen einer linken Tibia die linke Seite bzw. Hälfte des Patientenkörpers und/oder das linke Bein des Patienten als zutreffende Körperregion bestimmt werden.

Der oben dargestellte Vergleich der Patienten-Landmarken mit den Modell-Landmarken ermöglicht es, die Zuordnung des anatomischen Körperteils zur Körperregion durchzuführen, ohne dass ein Benutzer eine unmittelbare (insbesondere manuelle) Zuordnung der Lagen von Patienten-Landmarken zu Lagen von Modell-Landmarken vornehmen muss. Vielmehr kann die Zuordnung automatisch erfolgen, da die Elemente des Satzes von Patienten-Landmarken gleich zu den Elementen des Satzes von Modell-Landmarken sind. Insbesondere bezeichnen die Elemente beider Sätze spiegelsymmetrische Koordinaten bzw. Lagen von Orten am anatomischen Körperteil bzw. im Patientenkörper. Die Spiegelsymmetrie liegt dabei vorzugsweise zu einer Spiegelebene bzw. Symmetrieebene in der Sagittalebene bzw. medianen Sagittalebene des Patientenkörpers und/oder des Modells des Patientenkörpers vor.

Die Information über das räumliche Muster der Lagen der Modell-Landmarken beschreibt insbesondere eine Lageverteilung, d.h. eine Verteilung der Lagen der Modell-Landmarken im Raum. Dieser Raum wird vorteilhaft durch ein patientenzentriertes Koordinatensystem beschrieben, d.h. ein Koordinatensystem, das relativ zum Patientenkörper definiert ist. Unter der Lageverteilung kann so z.B. ein (insbesondere räumliches) Muster bzw. eine geometrische Anordnung der Modell-Landmarken in einem solchen Koordinatensystem verstanden werden. Ein solches Muster kann dann mit einem Muster verglichen werden, das sich aus der räumlichen Anordnung der Patienten-Landmarken ergibt. Das Koordinatensystem kann kartesisch und/oder sphärisch sein. Analog können die Informationen über das räumliche Muster der Lagen der Patienten-Landmarken eine Lageverteilung der Patienten-Landmarken beschreiben. In diesem Fall sind die gleichen Varianten möglich wie eben für die Modell-Landmarken beschrieben.

Im Rahmen dieser Erfindung ist es auch möglich, eine anterior-posterior-Richtung des anatomischen Körperteils zu bestimmen. Unter einer anterior-posterior-Richtung wird insbesondere eine Richtung verstanden, die senkrecht zu einer Frontalebene des Patientenkörpers verläuft. Stellt der anatomische Körperteil einen Knochen in einer Gliedmaße des Patientenkörpers dar, so verläuft die anterior-posterior-Richtung ebenfalls senkrecht zur Längsachse dieses Knochens, falls die Längsachse in proximal-distal-Richtung verläuft. Die eindeutige Zuordnung der Lageverteilung der Patienten-Landmarken zu einer Körperregion, insbesondere einer rechten oder linken Körperseite kann mithilfe weiterer geometrischer Überlegungen auch zur Bestimmung einer anterior-posterior-Richtung des anatomischen Körperteils genutzt werden. Eine solche Richtungsbestimmung kann im chirurgischen Navigationsverfahren wichtig sein, um z.B. bestimmte Instrumente relativ zu dem anatomischen Körperteil navigieren zu können.

Voraussetzung dafür ist die Bestimmung der richtigen Körperregion, in der sich der anatomische Körperteil befindet. Beispielsweise kann hierzu die Lage einer weiteren Patienten-Landmarke bzw. Modell-Landmarke herangezogen werden, die in einer charakteristischen, insbesondere ausgezeichneten Lage relativ zu einer oder mehr weiteren Patienten-Landmarken bzw. Modell-Landmarke, vorteilhaft zwei weiteren Patienten-Landmarken bzw. Modell-Landmarken steht. Im Falle der Bestimmung einer Längsachse und/oder anterior-posterior-Achse einer Tibia kann beispielsweise die Patienten-Landmarke bzw. Modell-Landmarke auf dem Malleolus lateralis herangezogen werden, um eine solche eindeutige Zuordnung der referenzierten Patienten-Landmarken zur rechten oder linken Körperseite des Patienten bzw. zu seinem rechten oder linken Bein bzw. den äquivalenten Modell-Landmarken vorzunehmen. Ebenso könnte beispielsweise eine Landmarke auf dem Schulterblatt und/oder dem Schlüsselbein verwendet werden, um einen Oberarmknochen der rechten oder linken Körperhälfte des Patienten zuzuordnen. Erfindungsgemäß möglich ist es auch, eine Landmarke auf der Elle oder Speiche zu verwenden, die eine ausgezeichnete Lage gegenüber anderen Landmarken auf dem jeweils anderen Knochen (d.h. entweder Speicher oder Elle) aufweist, um den Knochen, der die anderen Landmarken umfasst, im rechten oder linken Arm bzw. der rechten oder linken Körperhälfte des Patienten zuzuordnen. Wird z.B. der Abstand der Patienten-Landmarke bzw. Modell-Landmarken auf dem Malleolus medialis zu einer der Patienten-Landmarken auf dem Condylus medialis und/oder Condylus lateralis und/oder der Eminentia intercondylaris mit dem Abstand der Patienten-Landmarke bzw. Modell-Landmarke auf dem Malleolus lateralis zu wenigstens einer der Patienten-Landmarken bzw. Modell-Landmarke auf dem Condylus medialis, Condylus lateralis oder der Eminentia intercondylaris sowie vorteilhaft der bekannten Lage einer Markervorrichtung verglichen, die in diesem Fall gewöhnlich auf der medialen Seite der Tuberositas tibiae an der Tibia angebracht wird, so ergibt sich eine eindeutige Zuordnung des anatomischen Körperteils (in diesem Fall des Unterschenkels) zum rechten oder linken Bein des Patienten. Erfindungsgemäß werden also vorteilhaft Abstände und/oder Richtungen zwischen Patienten-Landmarken und zwischen den entsprechenden Modell-Landmarken verglichen.

Bei bekannter Lage der Markervorrichtung kann auch auf die Bestimmung der Abstände vorteilhaft verzichtet werden, und eine Winkelgröße zwischen zwei charakteristischen Geraden bzw. Ebenen zur Bestimmung der Körperregion ausreichen. So kann beispielsweise durch die Lagen der Patienten-Landmarken auf dem Malleolus medialis und auf dem Malleolus lateralis eine Gerade bzw. Ebene gelegt werden (vorteilhaft durchquert diese Gerade die Koordinaten von wenigstens zwei Patienten-Landmarken) sowie eine Senkrechte gezogen werden, die senkrecht auf der Oberfläche bzw. Tangente der Oberfläche des Malleolus lateralis im Ort der Patienten-Landmarke auf dem Malleolus lateralis steht. Der von der Geraden bzw. Ebene und der Senkrechten eingeschlossene Winkel kann insbesondere zusammen mit Informationen über die Lage des Referenzpunkts genutzt werden, um eine eindeutige Zuordnung des Satzes von Patienten-Landmarken zu einer rechten oder linken Tibia vorzunehmen.

Alternativ kann eine solche Gerade auch durch die Patienten-Landmarke im Malleolus lateralis und eine Patienten-Landmarke gelegt werden, die nicht auf dem Malleolus medialis angeordnet ist, so z.B. die Patienten-Landmarke in der Eminentia intercondylaris.

Die eben beschriebene Bestimmung von Abständen und/oder Richtungen zwischen den Patienten-Landmarken bzw. Modell-Landmarken oder des Winkels zwischen der Geraden und der Senkrechten wird als Bestimmung von Lagekriterien bezeichnet, die von der zu bestimmenden Körperregion abhängig sind und die Lage der Patienten-Landmarken relativ zueinander beschreiben, so dass deren eindeutige Entsprechung im Satz von Modell-Landmarken identifiziert werden kann. Der Winkel bzw. die Abstände und/oder Richtungen stellen also Lagekriterien dar.

Aus der Information über die Körperregion, der der anatomische Körperteil zuzuordnen ist, kann auch eine Längsachse bzw. mechanische Achse des Körperteils ermittelt werden. Insbesondere ist dies möglich, wenn der anatomische Körperteil wenigstens einen Bestandteil einer Gliedmaße, z.B. eine Tibia darstellt. Wurde wie oben dargestellt der Winkel zwischen einer Geraden durch zwei Landmarken und einer Senkrechten auf die Oberfläche eines Körperteils, in der sich eine der beiden Patienten-Landmarken befindet, ermittelt, so ist die Richtung dieser Senkrechten bekannt. Vorteilhaft liegt auf dieser Senkrechten die Landmarke im Malleolus lateralis (die Senkrechte wird daher im Folgenden als Malleolus-Senkrechte bezeichnet). Dann kann eine zweite Senkrechte (die im Folgenden als Längs-Senkrechte bezeichnet wird) von der Eminentia intercondylaris auf die Malleolus-Senkrechte gefällt werden, so dass die Richtung der Längs-Senkrechte mit der Längsachse der Tibia zusammenfällt. Somit ist dann die Längsachse der Tibia bestimmt.

Ist wie oben dargestellt eine Malleolus-Senkrechte (also eine Senkrechte, die senkrecht zur Längsachse des Körperteils verläuft, also eine Quersenkrechte) bekannt und auch die Richtung der Längsachse des Körperteils bekannt, so kann eine dritte Richtung ermittelt werden, die senkrecht sowohl auf der Längsachse als auch der Quersenkrechten steht. Diese Richtung entspricht dann einer anterior-posterior-Richtung des anatomischen Körperteils. Im Übrigen kann die Längsachse der Tibia auch ohne Bestimmung der Quersenkrechten erfolgen, wenn der zu erwartende Abstand der Längsachse vom Referenzpunkt und/oder einem bekannten Ort auf einer Markervorrichtung (die vorteilhaft am Referenzpunkt angebracht ist) bekannt ist. So wird eine Gerade konstruiert, die durch die Landmarke auf der Eminentia intercondylaris und einen virtuellen Punkt verläuft, der eine bekannte Lage relativ zum Referenzpunkt und/oder relativ zu einem Ort auf einer Markervorrichtung hat. Vorteilhaft hat der virtuelle Punkt einen bekannten Abstand zum Referenzpunkt und/oder einem bekannten Punkt auf der Markervorrichtung. Somit lässt sich die Richtung der Längsachse als Richtung einer Geraden durch die Landmarke auf der Eminentia intercondylaris und den virtuellen Punkt mithilfe der bekannten Lage der Landmarke in der Eminentia intercondylaris und dem bekannten Abstand des Referenzpunkts und/oder einem bekannten Ort auf einer Markervorrichtung konstruieren.

Mithilfe der Information über die Lageverteilung der Patienten-Landmarken und gegebenenfalls des Referenzpunkts (bzw. einer Markervorrichtung) kann beispielsweise so auch ermittelt werden, ob der Benutzer eine Vorder- oder Rückseite (eine anteriore oder posteriore Seite) des Körpers bzw. anatomischen Körperteils (insbesondere im Operationsfeld) vor sich hat bzw. aus seiner Perspektive betrachtet.

Vorteilhaft ist das erfindungsgemäße Verfahren als Datenverarbeitungsverfahren bzw. medizindatentechnisches Verfahren implementiert. Das Datenverarbeitungsverfahren wird bevorzugt durch technische Mittel, insbesondere einem Computer, durchgeführt. Der Computer umfasst insbesondere einen Prozessor und insbesondere einen Speicher, um insbesondere elektronisch die Daten zu verarbeiten. Insbesondere werden die beschriebenen Schritte des Berechnens von einem Computer ausgeführt. Schritte des Definierens von z. B. Bereichen oder Werten sind insbesondere Schritte des Festlegens von Daten im Rahmen des technischen Datenverarbeitungsverfahrens, insbesondere im Rahmen eines Programms. Schritte des Änderns stellen insbesondere eine Änderung der Daten mittels des Computers dar. Schritte des Ermittelns umfassen insbesondere die Abfrage von Werten, die an einer Schnittstelle des Computers anliegen und die durch technische Mittel, wie z. B. einer Abtasteinrichtung, erzeugt wurden. Diese Werte werden insbesondere von der Schnittstelle in Daten umgewandelt, die von dem Computer verarbeitet werden können.

Im Rahmen der Erfindung können Computerprogrammelemente von Hardware und/oder Software (dies beinhaltet auch Firmware, im System abgespeicherte Software, Mikrocode usw.) verkörpert werden. Im Rahmen der Erfindung können Computerprogrammelemente die Form eines Computerprogrammprodukts annehmen, das durch ein auf einem Computer verwendbares oder computerlesbares Speichermedium verkörpert werden kann, das auf einem Computer verwendbare oder computerlesbare Programmanweisungen, "Code" oder ein "Computerprogramm" umfasst, die bzw. der bzw. das auf dem genannten Medium zur Verwendung auf oder in Verbindung mit dem System, das die Anweisung ausführt, verkörpert sind bzw. ist. Ein solches System kann ein Computer sein, ein Computer kann eine Datenverarbeitungsvorrichtung mit Mitteln zum Ausführen der Computerprogrammelemente und/oder des erfindungsgemäßen Programms sein. Im Rahmen dieser Erfindung kann ein auf einem Computer verwendbares oder computerlesbares Medium irgendein Medium sein, das das Programm zur Verwendung auf oder in Verbindung mit dem System, Apparat oder der Einrichtung, das bzw. der bzw. die die Anweisung ausführt, das Programm beinhalten, speichern, übermitteln, fortpflanzen oder transportieren kann. Das auf einem Computer verwendbare oder computerlesbare Medium kann z.B. ein elektronisches, magnetisches, optisches, elektromagnetisches, Infrarot- oder Halbleiter-System, ein solcher Apparat oder eine solche Einrichtung oder ein Ausbreitungsmedium wie z.B. das Internet sein, ist aber nicht auf diese Aufzählung beschränkt. Das auf einem Computer verwendbare oder computerlesbare Medium könnte sogar z.B. Papier oder ein anderes geeignetes Medium sein, auf das das Programm gedruckt ist, da das Programm elektronisch erfasst werden könnte durch z.B. optisches Scannen des Papiers oder anderen geeigneten Mediums und dann kompiliert, interpretiert oder andernfalls auf geeignete Weise prozessiert werden könnte. Das Computerprogrammprodukt und jegliche Software und/oder Hardware, das bzw. die hier beschrieben werden, bilden in den beispielhaften Ausführungsformen die verschiedenen Mittel zum Ausführen der Funktionen der Erfindung. Insbesondere kann der Computer bzw. die Datenverarbeitungsvorrichtung eine Hinweisinformationsvorrichtung beinhalten, die Mittel zum Ausgeben einer Hinweisinformation beinhaltet. Die Hinweisinformation kann visuell durch ein visuelles Anzeigemittel (z. B. einen Monitor und/oder eine Lampe) und/oder akustisch durch ein akustisches Anzeigemittel (z. B. einen Lautsprecher und/oder eine digitale Sprachausgabeeinrichtung) und/oder taktil durch ein taktiles Anzeigemittel (z. B. ein in ein Instrument eingebautes vibrierendes Element bzw. Vibrationselement) beispielsweise an einen Benutzer ausgegeben werden.

Vorteilhaft gibt die Hinweisinformationsvorrichtung Hinweisinformationen (insbesondere Hinweissignale) an einen Benutzer aus, die auf der Bestimmung der Patienten-Körperregion basieren. So kann dem Benutzer mit dem visuellen Anzeigemittel und/oder dem akustischen Anzeigemittel (z.B. durch eine Textausgabe und/oder eine Sprachmodulausgabe) und/oder taktile Anzeigemittel (z.B. Vibrationsmittel) mitgeteilt werden, welcher Region des Patientenkörpers der anatomische Körperteil zuzuordnen ist, so z.B. ob es sich um eine rechte oder linke Tibia handelt.

Teil der Erfindung ist auch ein computerlesbares Medium, auf dem ein Programm bzw. Datenverarbeitungsverfahren gespeichert ist, das das oben dargestellte Verfahren ausführt, wenn es in eine Datenverarbeitungsvorrichtung geladen wird und auf dieser abläuft. Die Datenverarbeitungsvorrichtung ist insbesondere ein handelsüblicher Computer und/oder der Computer eines chirurgischen Navigationssystems.

Ebenfalls Bestandteil dieser Erfindung ist ein System zum Bestimmen der Körperregion, in der sich ein anatomischer Körperteil befindet. Ein solches System umfasst vorteilhaft einen Pointer zum Bestimmen der anatomischen Punktdaten, ein computerlesbares Speichermedium, auf dem die Landmarken-Daten gespeichert sind, sowie ein chirurgisches Navigationssystem zum Vergleichen der anatomischen Punktdaten mit den Landmarker-Daten. Vorteilhaft ist das chirurgische Navigationssystem bzw. die Datenverarbeitungsvorrichtung, auf der das erfindungsgemäße Verfahren abläuft, so konfiguriert, dass es bzw. sie basierend auf der Annahme, dass die Patienten-Landmarken die gleichen Landmarken darstellen wie die Modell-Landmarken und gegebenenfalls dass der Referenzpunkt in einer Lagebeziehung zu den Patienten-Landmarken steht, die vergleichbar der Lagebeziehung des Referenzpunkts zu den Modell-Landmarken ist (sofern der Referenzpunkt notwendig zur Seitenbestimmung ist), die Körperregion bestimmen, auf der sich der Patienten-Körperteil befindet. Die Bestimmung der Körperregion basiert dann vorteilhaft auf einem Vergleich der Landmarken-Daten und der anatomischen Punktdaten wie oben dargestellt.

Das System zum Bestimmen der Körperregion kann auch das oben beschriebene computerlesbare Speichermedium sowie die Hinweisinformationsvorrichtung zur Ausgabe von Hinweisinformationen umfassen. Dabei weisen die Hinweisinformationen vorteilhaft auf die ermittelte Körperregion hin, der der anatomische Körperteil zugeordnet wurde.
Figur 1 zeigt eine Tibia und eine Fibula mit einer Markervorrichtung sowie einem chirurgischen Navigationssystem.
Figur 1a zeigt schematisch den Vergleich der Lageverteilung der Patienten-Landmarken mit den Lageverteilungen zweier achsensymmetrisch zueinander gelegener Sätze von Modell-Landmarken.
Figur 1b zeigt die Symmetrieverhältnisse zwischen zwei seitenverschiedenen Sätzen von Modell-Landmarken.
Figur 2 zeigt eine axiale Ansicht einer Tibia mit Kennzeichnung einer anterior-posterior-Richtung.
Figur 3 zeigt die Landmarken im Condylus medialis und Condylus lateralis.
Figur 4 zeigt ein Ablaufdiagramm des erfindungsgemäßen Verfahrens.

Das Bezugszeichen X steht im Folgenden als Abkürzung für den Bezugszeichensatz 1a, 3, 4, 5, 6, 7. Das Bezugszeichen Y steht im Folgenden als Abkürzung für den Bezugszeichensatz 17, 18, 20, 21, 22, 23.

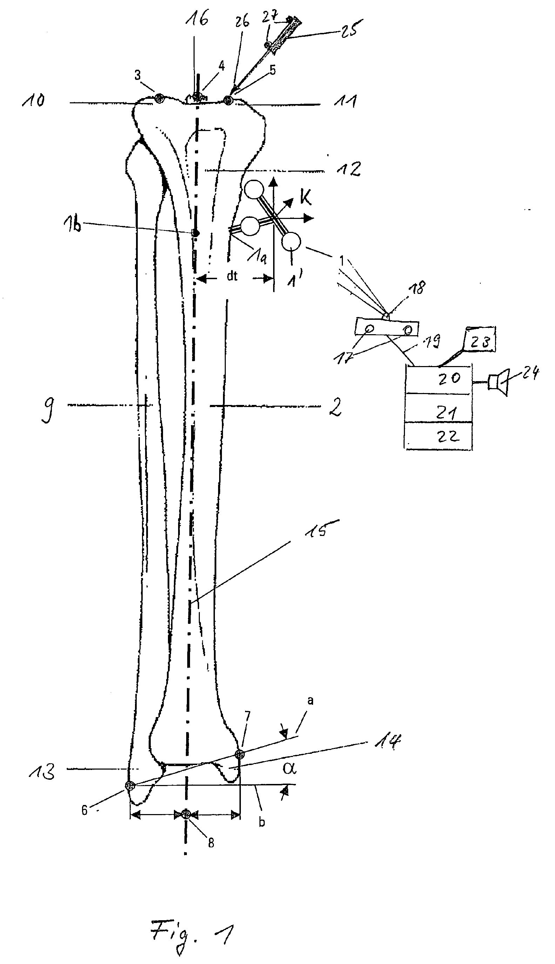

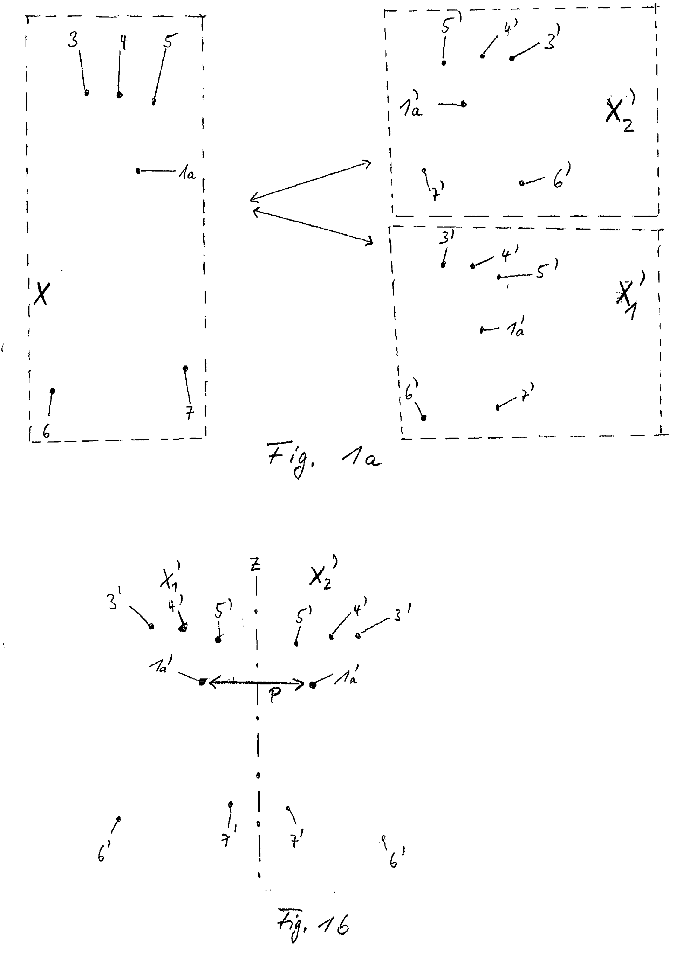

Figur 1 zeigt die Lage der Patienten-Landmarken X auf einer Tibia und einer Fibula sowie die Ermittlung der Tibiaachse (Längsachse) mithilfe eines chirurgischen Navigationssystems. Auf einer Tibia 2 ist ein Referenzstern 1 im Referenzpunkt 1a im Bereich der Tuberositas tibiae 12 angebracht. Auf den Referenzstern 1 kann auch verzichtet werden, wenn zu Beginn des Verfahrens der Referenzpunkt 1a mithilfe eines Pointers 25 registriert bzw. festgelegt wird oder wenn wenigstens eine Patienten-Landmarke X nicht in einer gemeinsamen Ebene mit den anderen Patienten-Landmarken X liegt. Die Registrierung beinhaltet die Zuordnung des Referenzpunkts 1 im Patientendatensatz zu den entsprechenden Koordinaten im Modell-Datensatz. Mithilfe des Pointers 25, der Markerkugeln 27 und eine Pointerspitze 26 umfasst, kann der Operateur die Patienten-Landmarken X abgreifen. Dadurch ermöglicht der Benutzer einem chirurgischen Navigationssystem 17, 18, 20, 21, 22, 23 die Erfassung der Koordinaten bzw. Lagen der Patienten-Landmarken X relativ zu einem Koordinatensystem K. Das Koordinatensystem K kann fest bezüglich des Patientenkörpers und/oder des anatomischen Körperteils (beispielsweise der Tibia 2) sein. Das Koordinatensystem K kann aber auch fest zu einem Ursprung sein, der sich außerhalb des Patientenkörpers, beispielsweise im Standort des chirurgischen Navigationssystems Y befindet.

Das chirurgische Navigationssystem umfasst einen Sender 18 für Infrarotstrahlung und zwei Empfänger (insbesondere eine stereotaktische Kamera) 17, die für Infrarotstrahlung empfindlich sind. Eine Datenleitung 19 übermittelt Signale zwischen einem Prozessor 20 und dem Sender 18 sowie dem Empfänger 17. An dem Prozessor 20 sind ein Monitor 23 sowie eine Hinweisinformationsvorrichtung 24 zur Ausgabe von hörbaren und/oder sehbaren Hinweisinformationen an einen Benutzer angeschlossen. Die Hinweisinformationsvorrichtung 24 umfasst vorteilhaft einen Lautsprecher und kann in den Monitor 23 integriert sein. Ebenfalls an den Prozessor 20 angeschlossen sind ein Arbeitsspeicher 21 sowie ein Permanentspeicher 22 (beispielsweise eine Festplatte oder ein anderes magnetisches und/oder optisches computerlesbares Speichermedium). Auf dem Permanentspeicher 22 können die Landmarken-Daten sowie die Lagekriterien (α) gespeichert sein.

Der Benutzer kann nun mit dem Pointer 25 Patienten-Landmarken X abgreifen. Vorteilhaft sind diese Landmarken wie folgt angeordnet: Eine Landmarke 3 befindet sich auf dem Condylus lateralis 10, eine Landmarke 4 befindet sich auf der Eminentia intercondylaris 16, eine Landmarke 5 befindet sich auf dem Condylus medialis 11, eine Landmarke 6 befindet sich auf Malleolus lateralis 13, eine Landmarke 7 befindet sich auf dem Malleolus medialis 14. Damit wird klar, dass auch eine Landmarke (nämlich die Landmarke 6) auf der Fibula 9 angeordnet ist. Das chirurgische Navigationssystem Y liest nun die anatomischen Punktdaten ein und ermittelt daraus eine Lageverteilung der Lagen von Patienten-Landmarken X. Ferner kann dem chirurgischen Navigationssystem Y die Lage des Referenzpunkts 1a bzw. des Referenzsterns 1 relativ zur Tibia 2 bekannt sein. Vorteilhaft liegt der Referenzpunkt 1a bzw. der Ort, an dem der Referenzstern 1 befestigt ist, im Bereich der Tuberositas tibiae 12. Da auch ohne Verwendung des Referenzstems 1 der Referenzpunkt 1a mit dem Pointer 25 abgegriffen werden kann, ist dem chirurgischen Navigationssystem Y die Lage des Referenzpunkts 1 a relativ zur Tibia 2 sowohl mit als auch ohne Verwendung des Referenzsterns 1 bekannt.

Figur 1a zeigt einen Vergleich des Satzes von Patienten-Landmarken X mit zwei Sätzen von Modell-Landmarken X₁', X₂' ein, wobei jeweils ein Satz aus Modell-Landmarken besteht, die dem Modell des anatomischen Körperteils auf einer bestimmten Körperseite entsprechen. Der Satz X₁' umfasst dabei Modell-Landmarken 3', 4', 5', 6', 7' sowie einen Referenzpunkt 1a' (der auch als Modell-Landmarke angesehen werden kann); ein Satz X₂' umfasst dabei die entsprechenden Modell-Landmarken für den Körperteil, der im Modell spiegelsymmetrisch bezüglich einer Sagittalebene (insbesondere der medianen Sagittalebene) des Modells des Patientenkörpers zum Satz Modell-Landmarken X₁' gelegen ist. Stellt der Satz Patienten-Landmarken X bezüglich des Referenzpunktes 1a beispielsweise die geometrischen Verhältnisse an einem rechten Unterschenkel des Patienten dar, so ergibt ein Vergleich mit den Sätzen von Modell-Landmarken X₁'und X₂' (deren Lage relativ zu Referenzpunkten 1a' definiert ist), dass die Anordnung der Patienten-Landmarken X mit einer höheren Wahrscheinlichkeit der Anordnung der Modell-Landmarken X₁' entspricht als der Anordnung der Modell-Landmarken X₂'.

Dies wird umso klarer, wenn man Figur 1b betrachtet: Hier sind die Symmetrieverhältnisse zwischen den Sätzen Modell-Landmarken X₁'und X₂' bezüglich einer Achse z, die in der medianen Sagittalebene des Patienten liegt, dargestellt. Werden nun die Patienten-Landmarken X in der Reihenfolge ihrer Nummerierung abgegriffen, so ergibt sich insbesondere bezüglich der Lage des Referenzpunkts 1a eine charakteristische Verteilung der Patienten-Landmarken X. Diese Verteilung beinhaltet insbesondere Informationen über die Abstände der Patienten-Landmarken X zueinander sowie vorteilhaft Informationen über Winkel (vorzugsweise ebene und/oder räumliche Winkel) zwischen die einzelnen Patienten-Landmarken X miteinander verbindenden Geraden. Gleiche Informationen liegen über die Sätze Modell-Landmarken X₁' und X₂' vor. Für jeden der Sätze Modell-Landmarken X₁' und X₂' können ferner Informationen vorliegen, in welcher Reihenfolge die Lagen der Modell-Landmarken relativ zum Referenzpunkt 1a' mit den Lagen der Patienten-Landmarken X zum Referenzpunkt 1a verglichen werden sollen. Aus Figur 1b geht hervor, dass die Lagen der Modell-Landmarken relativ zum Referenzpunkt 1a' unterschiedlich bezüglich einer Symmetrieachse z in der Sagittalebene des Modells des Patientenkörpers sind. Aufgrund dieses Unterschiedes kann im Vergleich mit den Lagen der Patienten-Landmarken X relativ zum Referenzpunkt 1a ermittelt werden, mit welchem Satz Modell-Landmarken X₁', X₂' der Satz Patienten-Landmarken X übereinstimmt. Als Übereinstimmung wird in diesem Zusammenhang insbesondere eine geometrische Ähnlichkeit der Lageverteilungen zwischen dem Satz Patienten-Landmarken X und den Sätzen Modell-Landmarken X₁' und X₂' bezeichnet. Diese Ähnlichkeit gründet auf den oben beschriebenen Vergleich von Abständen und/oder Winkeln bzw. Richtungen. Vorteilhaft lässt das erfindungsgemäße Verfahren jedoch Abweichungen zwischen den Lageverteilungen im Satz Patienten-Landmarken X und dem zutreffenden (d.h. zu diesem insbesondere geometrisch ähnlichen) Satz Modell-Landmarken X₁', X₂' zu, so dass selbst bei vorliegen dieser Abweichung eine Übereinstimmung festgestellt werden kann. Eine solche Abweichung kann insbesondere ein relativer Fehler zwischen den Positionen der Landmarken sein, der beispielsweise einen Wert von 10⁻² oder auch 10⁻³ hat und/oder in einem Intervall liegt, dessen Obergrenze bei 10⁻¹ oder 10⁻² oder 10⁻³ liegt und dessen Untergrenze bei 10⁻² oder 10⁻³ oder 10⁻⁴ liegt. Erfindungsgemäß ist auch die Definition einer absoluten Abweichung möglich, die beispielsweise 1 µm, 1 mm oder 5 mm betragen kann oder in einem Intervall liegen kann, dessen Obergrenze vorteilhaft bei 5 mm oder 1 mm liegt und dessen Untergrenze bei vorteilhaft 1 mm oder 1 µm liegen kann. In Figur 1b zeigt ferner der Doppelpfeil P anschaulich die achsensymmetrische Lage der Bezugspunkte 1a' bezüglich einer Symmetrieachse z der beispielsweise rechten (Modell-Landmarken X₁') und linken (Modell-Landmarken X₂') unteren Extremität eines Modells des Patientenkörpers. Insbesondere ist der Referenzpunkt 1a' auf den beiden symmetrischen Körperteilen an einander entsprechenden Orten (d.h. jeweils in der gleichen Lage relativ zur Tuberositas tibiae 12) angeordnet.

Das chirurgische Navigationssystem Y kann die Lage einer Geraden a durch die Patienten-Landmarken 7 und 6 sowie die Lage einer Senkrechten b auf eine Tangentialebene durch die Oberfläche des Malleolus lateralis 13 im Punkt der Patienten-Landmarke 6 errechnen. Daraus wiederum ermittelt das chirurgische Navigationssystem Y den Wert des Winkels α, der von der Gerade a und der Quersenkrechten b eingeschlossen wird. Dieser Winkel α wird nun mit einem vorbekannten Wert dieses Winkels für die Lageverteilung der Landmarken X verglichen. Beispielsweise dann, wenn das Ergebnis ist, dass die Landmarke 6 sich weiter vom Referenzpunkt 1a entfernt befindet als die Landmarke 7, kann davon ausgegangen werden, dass es sich um einen linken Unterschenkel handelt: Das Abgreifen der Patienten-Landmarken X wird vorzugsweise in der Reihenfolge ihrer bezugszeichenmäßigen Nummerierung bzw. in einer zuvor festgelegten Reihenfolge beginnend insbesondere beim Referenzpunkt 1a durchgeführt, so dass die Lage der Patienten-Landmarke 6 beispielsweise relativ zum Referenzpunkt 1a und zur Patienten-Landmarke 7 charakteristisch für die rechte oder linke Seite des Patientenkörpers ist.

Ferner kann beispielsweise das chirurgische Navigationssystem Y (bzw. dessen Prozessor 20) eine Gerade 15 berechnen, die sowohl senkrecht auf der Quersenkrechten b steht als auch durch die Patienten-Landmarke 4 auf der Eminentia intercondylaris 16 geht. Die Gerade 15 fällt somit insbesondere mit der Längsachse der Tibia bzw. einer mittleren Tibiaachse in Längsrichtung der Tibia zusammen. Die Längsachse verläuft dabei vorteilhaft von der Eminentia intercondylaris in Richtung des distalen Endes der Tibia. Ferner ist bekannt, dass diese Gerade die Projektion der Geraden a auf die Quersenkrechte b genau in zwei gleichgroße Teile teilt. Der Abstand eines Punkts 8 von einer Tangente an die Oberfläche des Malleolus medialis im Ort der Patienten-Landmarke 7 ist gleich dem Abstand des Punkts 8 zu einer Tangente an die Oberfläche des Malleolus lateralis im Ort der Patienten-Landmarke 6; der Punkt 8 kann somit als Mittelpunkt des Abstands zwischen den Landmarken 6 und 7 angesehen werden. Mit diesen Informationen lässt sich die Lage der Tibiaachse 15 bzw. der Längsachse 15 der Tibia konstruieren bzw. errechnen, indem eine Gerade 15 durch den Punkt 8 und die Landmarke 4 gelegt wird. Ferner kann auch bekannt sein, dass die Tibiaachse 15 beispielsweise in einem Punkt 1b, der vorteilhaft zwischen dem Punkt 8 und der Landmarke 4 liegt, einen bestimmten Abstand dt von einem Bestandteil des Referenzsterns haben und parallel zu einer Achse des Koordinatensystems K liegen soll. Die betreffende Achse des Koordinatensystems K läuft dabei vorzugsweise parallel zur Sagittalebene des Patientenkörpers bzw. dessen Modell. Auch aus diesen beiden Informationen, die die Lage der Tibiaachse 15 relativ zum Referenzstern 1 beschreiben, kann die Tibiaachse 15 konstruiert werden.

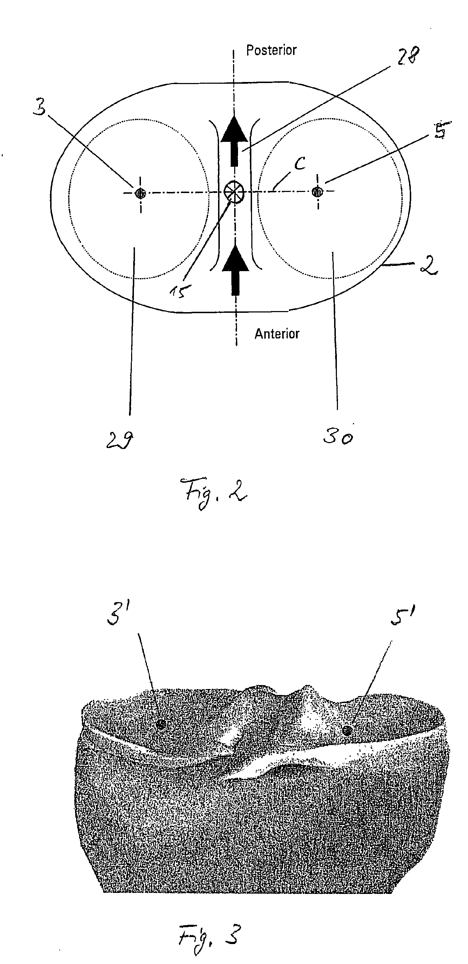

Figur 2 zeigt das Verhältnis einer anterior-posterior-Richtung 28 der Tibia 2 zur Richtung der Quersenkrechten b sowie der Richtung der Tibiaachse 15. Die Quersenkrechte b läuft parallel zu einer Geraden durch die beiden Patienten-Landmarken 3 und 5 die im lateralen Plateau 29 und medialen Plateau 30 der abgebildeten Tibia 2 liegen. Die Tibia 2 stellt insbesondere eine rechte Tibia in anteriorer Ansicht dar. Figur 3 verdeutlicht die dreidimensionale Lage der Patienten-Landmarken 3 und 5 in Gestalt der Modell-Landmarken 3' und 5' auf den Condyli.

Da die Gerade c senkrecht zur Richtung der Tibiaachse 15 und vorzugsweise parallel zur Quer-Senkrechten b verläuft, kann das chirurgische Navigationssystem Y (bzw. dessen Prozessor 20) aus der Kenntnis der Richtung der Quersenkrechten b und der Tibiaachse 15 eine auf beiden Richtungen senkrecht stehende Ebene berechnen, in der die anterior-posterior-Richtung 28 (a-p-Richtung 28) liegt. Somit kann dem Benutzer auch eine Hinweisinformation darüber mittels der Hinweisinformationsvorrichtung 26 ausgegeben werden, ob er ein mit einer Markervorrichtung versehenes Instrument gerade auf der anterioren oder posterioren Seite der Tibia 2 anwendet.

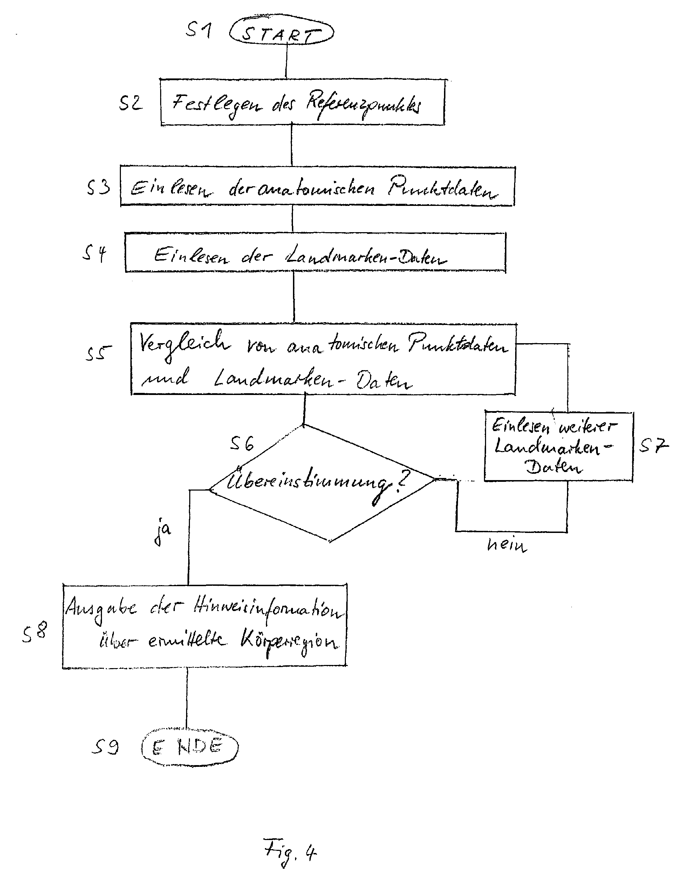

Figur 4 zeigt ein Ablaufdiagramm für das erfindungsgemäße Verfahren, bei dem der Vergleich der anatomischen Punktdaten mit den Landmarken-Daten effizient gestaltet ist. Das Verfahren ist zur Ausführung auf einer Datenverarbeitungsvorrichtung 20, 21, 22 vorgesehen, besitzt mithin die Struktur eines Datenverarbeitungsprogramms. Das Programm startet in einem Schritt S 1 mit einem Aufruf durch den Prozessor 20. Vorteilhaft wird in einem Schritt S2 der Referenzpunkt festgelegt, indem das chirurgische Navigationssystem die Lage des Referenzpunkts 1a infolge Abgreifens mit einem Pointer 25 oder aus der erfassten Lage eines Referenzstems 1 ermittelt. Falls ein Referenzstern 1 zur Anwendung kommt, ist bekannt, welche Lage der Referenzpunkt 1a relativ zu den Markern 1' des Referenzstems 1 aufweist. In einem Schritt S3 liest das chirurgische Navigationssystem Y die anatomischen Punktdaten und damit die Lagen der Patienten-Landmarken X insbesondere relativ zum Referenzpunkt 1a vorteilhaft mit Hilfe des Abgreifens mit einem Pointer 25 ein. In einem Schritt S4 liest der Prozessor 20 die Landmarkendaten vorteilhaft aus dem Permanentspeicher 22 ein. Insbesondere kann der Benutzer vor Beginn des Verfahrens wählen, welcher anatomische Körperteil 2, 9 zur Behandlung im Rahmen des Verfahrens vorgesehen ist. Das Programm kann somit darauf abgestimmt sein, zuerst nur einen Satz Modell-Landmarken einzulesen, der der Anordnung des anatomischen Körperteils 2, 9 in einer bestimmten Körperregion (z.B. auf der linken Körperseite), die dem Programm mitgeteilt wurde, entspricht. Das vermeidet das Einlesen von Landmarken-Daten, die im anderen als in der betrachteten Körperregion liegende Landmarke betreffen. In einem Schritt S5 wird nun eine Lageverteilung der Patienten-Landmarken X (bzw. der anatomischen Punktdaten) mit einer Lageverteilung X₁', X₂' der Modell-Landmarken 1'a, 3', 4', 5', 6', 7' (bzw. der Landmarken-Daten) verglichen und in einem Schritt S6 wird das Ergebnis dieses Vergleichs bezüglich einer Übereinstimmung der Datensätze vorgenommen. Eine Überstimmung der Datensätze bedeutet in diesem Zusammenhang, dass die Lageverteilung der Patienten-Landmarken X der Lageverteilung X₁', X₂' der Modell-Landmarken 1a', 3', 4', 5', 6', 7', die in Form der Landmarken-Daten in Schritt S4 eingelesen worden sind, entspricht. Diese Entsprechung kann mit den oben dargestellten Kriterien für die Übereinstimmung der Lageverteilung in einem Satz Patient-Landmarken X und einem Satz Modell-Landmarken X₁', X₂' ermittelt werden. Ist eine Übereinstimmung gegeben, kann in einem Schritt S8 eine Ausgabe einer Hinweisinformation an den Benutzer erfolgen, mit der er in Kenntnis gesetzt wird, welcher Körperregion der anatomische Körperteil 2, 9 zuzuordnen ist. Diese Hinweisinformation kann beispielsweise als Audioinformation über die Hinweisinformationsvorrichtung 24 ausgegeben werden, erfindungsgemäß möglich ist aber auch die Ausgabe einer graphischen Information (z.B. durch farbliches Hervorheben der ermittelten Körperregion in einer graphischen Darstellung des menschlichen Körpers auf dem Monitor 23) oder in Form einer Textausgabe auf dem Monitor 23. Ergibt Schritt S6 jedoch keine Übereinstimmung, so wird erst in einem Schritt S7 der zweite aufgrund seiner Symmetrie grundsätzlich (d.h. aufgrund eines Operationsplans) zutreffende Satz Modell-Landmarken 1a', 3', 4', 5', 6', 7' eingelesen und einen Vergleich mit den anatomischen Punktdaten in Schritt S5 zugeführt. Dies erspart das Einlesen mit Sicherheit nicht zum gewünschten Ergebnis einer Übereinstimmung in S6 führender Daten. Es kann aber auch auf Schritt S7 verzichtet werden und aus dem Fehlen einer Übereinstimmung in Schritt S6 geschlossen werden, dass der Körperteil 2, 9 zu einer anderen bestimmten Körperregion zuzuordnen ist. Diese Vorgehen ist im Falle einer Tibia 2 gerechtfertigt, denn wenn beispielsweise die Lageverteilung der Patienten-Landmarken X beim Vergleich mit Modell-Landmarken 1'a, 3', 4', 5', 6', 7' einer linken Tibia des Patienten in Schritt S5 keine Übereinstimmung in Schritt S6 bewirkt, so muss notwendigerweise die Tibia 2 die rechte Tibia des Patienten darstellen. Dadurch kann auch eine Bereitstellung von Patientendaten mit einem bildgebenden Verfahren des jeweils für die Operation nicht zutreffenden zweiten, vorteilhaft symmetrisch (insbesondere spiegelsymmetrisch bezüglich der medianen Sagittalebene des Patientenkörpers bzw. des Modells des Patientenkörpers ) gelegenen anatomischen Körperteils vermieden werden. Wird der Schritt S7 jedoch in das erfindungsgemäße Verfahren einbezogen, so kann in Schritt S5 ein Positivtest über die Übereinstimmung der Lageverteilung von Patienten-Landmarken X mit der Lageverteilung X₁', X₂' von Modell-Landmarken 1a', 3', 4', 5', 6', 7' durchgeführt werden. Damit erhält der Benutzer eine Bestätigung über die Richtigkeit seines Vorgehens. In einem Schritt S9 endet das Programm, und die Information über die Übereinstimmung aus Schritt S6 und die zutreffende Körperregion kann vom Prozessor 20 im Permanentspeicher 22 oder im Arbeitsspeicher 21 abgelegt und für das weitere chirurgische Navigationsverfahren verwendet werden.

### Bezugszeichenliste

- 1: Referenzstern
- 1a: Referenzpunkt am Patienten-Körperteil (2, 9)
- 1a': Referenzpunkt im Modell des anatomischen Körperteils
- 2: Tibia
- 3: Patienten-Landmarke auf dem Condylus lateralis 10
- 3': Modell-Landmarke auf dem Condylus lateralis 10
- 4: Patienten-Landmarke auf der Eminentia intercondylaris 16
- 4': Modell- Landmarke auf der Eminentia intercondylaris 16
- 5: Patienten-Landmarke auf dem Condylus medialis 11
- 5': Modell-Landmarke auf dem Condylus medialis 11
- 6: Patienten-Landmarke auf dem Malleolus lateralis 13
- 6': Modell- Landmarke auf dem Malleolus lateralis 13
- 7: Patienten-Landmarke auf dem Malleolus medialis 14
- 7': Modell-Landmarke auf dem Malleolus medialis 14
- 8: Abstand der Landmarken 6, 7 auf den Malleoli lateralis 13 und medialis 14 von der Tibiachse 15
- 9: Fibula
- 10: Condylus lateralis
- 11: Condylus medialis
- 12: Tuberositas tibiae
- 13: Malleolus lateralis
- 14: Malleolus medialis
- 15: Tibiaachse
- 16: Eminentia intercondylaris
- 17: Empfänger
- 18: Sender
- 19: Datenleitung
- 20: Prozessor
- 21: Arbeitsspeicher
- 22: Permanentspeicher
- 23: Monitor
- 24: Hinweisinformationsvorrichtung
- 25: Pointer
- 26: Pointerspitze
- 27: Pointermarker
- 28: a-p-Richtung
- 19: laterales Plateau
- 30: mediales Plateau
- S1: Programmstart
- S2: Festlegen des Referenzpunkts
- S3: Einlesen der anatomischen Punktdaten
- S4: Einlesen der Landmarken-Daten
- S5: Vergleich
- S6: Prüfung auf Übereinstimmung
- S7: Einlesen weiterer Landmarken-Daten
- S8: Hinweisinformation
- S9: Programmende

## Patentansprüche

1. Verfahren zum Bestimmen der Körperregion, in der sich ein anatomischer Körperteil (2, 9) befindet, das folgende Schritte umfasst:
• anatomische Punktdaten, die Informationen über ein räumliches Muster von Lagen von Patienten-Landmarken (1a, 3, 4, 5, 6, 7) eines Patienten-Körperteils der dem anatomischen Körperteil (2, 9) entspricht, umfassen, werden bereitgestellt (S3), wobei sich die besagten Patienten-Landmarken an besagtem Patienten-Körperteil befinden,
• Landmarken-Daten werden bereitgestellt (S4, S7), die Informationen über ein räumliches Muster von Lagen von Modell-Landmarken (1a', 3', 4', 5', 6', 7') definiert in einem Modell des anatomischen Körperteils (2, 9) des Patienten umfassen;
wobei unter der Annahme,
• dass die Patienten-Landmarken (1a, 3, 4, 5, 6, 7) die gleichen Landmarken darstellen wie die Modell-Landmarken (1a', 3', 4', 5', 6', 7') und einander eindeutige zugeordnet werden können,
basierend auf den Landmarken-Daten und den anatomischen Punktdaten die Körperregion bestimmt wird, in der sich der Patienten-Körperteil befindet,
**dadurch gekennzeichnet, dass** die Patienten-Landmarken (1a, 3, 4, 5, 6, 7) eine künstliche Landmarke (1a) an dem Patienten-Körperteil und anatomische Landmarken (3, 4, 5, 6, 7) an dem Patienten-Körperteil umfassen und die Modell-Landmarken (1a', 3', 4', 5', 6', 7') eine künstliche Landmarke (1a') in dem Modell und anatomische Landmarken (3', 4', 5', 6', 7') in dem Modell umfassen.

2. Verfahren nach dem vorhergehenden Anspruch, bei dem die Lage der künstlichen Landmarke der Lage eines Referenzpunkts (1a) entspricht, wobei die Lage des Referenzpunkts (1a) insbesondere der Lage einer Körperteil-Markervorrichtung (1) entspricht.

3. Verfahren nach einem der vorhergehenden Ansprüche, wobei die Bereitstellung (S3) der anatomischen Punktdaten so erfolgt, dass keine manuelle Zuordnung der Lagen von Patienten-Landmarken (1a, 3, 4, 5, 6, 7) zu Lagen von Modell-Landmarken (1a', 3', 4', 5', 6', 7') vorgenommen wird, wobei die Elemente des Satzes von Patienten-Landmarken (1a, 3, 4, 5, 6, 7) gleich zu den Elementen des Satzes von Modell-Landmarken (1a', 3', 4', 5', 6', 7') sind.

4. Verfahren nach einem der vorhergehenden Ansprüche, wobei die Informationen über das räumliche Muster der Lagen der Modell-Landmarken (1a', 3', 4', 5', 6', 7') eine Lageverteilung (X₁', X₂') der Modell-Landmarken (1a', 3', 4', 5', 6', 7) beschreiben und die Informationen über das räumliche Muster der Lagen der Patienten-Landmarken (1a, 3, 4, 5, 6, 7) eine Lageverteilung (X₁, X₂) der Patienten-Landmarken (1a, 3, 4, 5, 6, 7) beschreiben.

5. Verfahren nach einem der vorhergehenden Ansprüche, wobei Lagekriterien bereitgestellt werden, die von der Körperregion abhängige Kriterien für die Lage der Patienten-Landmarken (1a, 3, 4, 5, 6, 7) zueinander und/oder der Modell-Landmarken (3', 4', 5', 6', 7) zueinander beschreiben.

6. Verfahren nach einem der vorhergehenden Ansprüche, bei dem die Bestimmung (S6) der Körperregion die Bestimmung eines Winkels (α) zwischen einer Geraden (a) durch wenigstens zwei bestimmte Patienten-Landmarken (6, 7) und/oder Modell-Landmarken (6', 7') und einer Senkrechten (b) zu einem Oberflächenausschnitt des Patienten-Körperteils umfasst, wobei die Lage einer der bestimmten Patienten-Landmarken (6, 7) und/oder Modell-Landmarken (6', 7') den Oberflächenausschnitt umfasst.

7. Verfahren nach einem der vorhergehenden Ansprüche, wobei bestimmt wird, dass die Körperregion eine linke oder rechte Körperseite ist.

8. Verfahren nach einem der vorhergehenden Ansprüche, bei dem Informationen über eine anterior-posterior-(a-p-) Richtung (28) des Patienten-Körperteils bestimmt werden.

9. Verfahren nach einem der vorhergehenden Ansprüche, wobei bestimmt wird, dass die Körperregion eine anteriore oder posteriore Körperseite ist.

10. Verfahren nach einem der vorhergehenden Ansprüche, wobei Informationen über die Lage einer Tibia-Achse (15) bestimmt werden.

11. Verfahren nach einem der vorhergehenden Ansprüche, bei dem basierend auf der Bestimmung (S6) der Patienten-Körperregion Hinweisinformationen an einen Benutzer ausgegeben werden (S8).

12. Programm, das, wenn es auf einem Computer geladen ist, den Computer veranlasst, die Schritte des Verfahrens nach einem der vorhergehenden Ansprüche auszuführen.

13. Navigationssystem eingerichtet zum Bestimmen der Körperregion, in der sich ein anatomischer Körperteil (2, 9) befindet, das folgendes umfasst:
• einen Pointer (25) zum Bestimmen von anatomischen Punktdaten, die Informationen über ein räumliches Muster von Lagen von Patienten-Landmarken (1a, 3, 4, 5, 6, 7) eines Patienten-Körperteils, der dem anatomischen Körperteil (2, 9) entspricht, umfassen;
• ein computerlesbares Speichermedium (22), auf dem Landmarken-Daten gespeichert sind, die Informationen über ein räumliches Muster von Lagen von Modell-Landmarken (1a', 3', 4', 5', 6', 7') eines Modells des anatomischen Körperteils (2, 9) des Patienten umfassen;
• ein chirurgisches Navigationssystem (17, 18, 20, 21, 22, 23) zum Vergleichen (S6) der anatomischen Punktdaten mit den Landmarken-Daten,
wobei das chirurgische Navigationssystem (17, 18, 20, 21, 22, 23) eine Datenverarbeitungsvorrichtung (20, 21, 22) umfasst, die konfiguriert ist, das Programm nach Anspruch 12 auszuführen.

14. Navigationssystem nach dem vorhergehenden Anspruch, das ein computerlesbares Speichermedium (22) umfasst, auf dem das Programm nach Anspruch 12 gespeichert ist.

15. Navigationssystem nach einem der beiden vorhergehenden Ansprüche, das eine Hinweisinformationsvorrichtung (23, 24) zur Ausgabe (S8) von Hinweisinformationen, die auf dem Vergleich der Landmarken-Daten und der anatomischen Punktdaten basieren, beinhaltet.

## Claims

1. A method for determining the region of the body in which an anatomical part of the body (2, 9) is situated, said method including the following steps:
• anatomical point data are provided (S3) which include information concerning a spatial pattern of positions of patient landmarks (1a, 3, 4, 5, 6, 7) of a part of a patient's body which corresponds to the anatomical part of the body (2, 9), wherein said patient landmarks are situated on said part of the patient's body;
• landmark data are provided (S4, S7) which include information concerning a spatial pattern of positions of model landmarks (1a', 3', 4', 5', 6', 7') defined in a model of the anatomical part of the patient's body (2, 9);
wherein, assuming that
• the patient landmarks (1a, 3, 4, 5, 6, 7) represent the same landmarks as the model landmarks (1a', 3', 4', 5', 6', 7') and can be clearly assigned to each other,
the region of the body in which the part of the patient's body is situated is determined on the basis of the landmark data and the anatomical point data,
**characterised in that** the patient landmarks (1a, 3, 4, 5, 6, 7) include an artificial landmark (1a) on the part of the patient's body and anatomical landmarks (3, 4, 5, 6, 7) on the part of the patient's body, and the model landmarks (1a', 3', 4', 5', 6', 7') include an artificial landmark (1a') in the model and anatomical landmarks (3', 4', 5', 6', 7') in the model.

2. The method according to the preceding claim, wherein the position of the artificial landmark corresponds to the position of a reference point (1a), wherein the position of the reference point (1a) in particular corresponds to the position of a marker device (1) on the part of the body.

3. The method according to any one of the preceding claims, wherein the anatomical point data are provided (S3) in such a way that the positions of patient landmarks (1a, 3, 4, 5, 6, 7) are not manually assigned to positions of model landmarks (1a', 3', 4', 5', 6', 7'), wherein the elements of the set of patient landmarks (1a, 3, 4, 5, 6, 7) are equal to the elements of the set of model landmarks (1a', 3', 4', 5', 6', 7').

4. The method according to any one of the preceding claims, wherein the information concerning the spatial pattern of the positions of the model landmarks (1a', 3', 4', 5', 6', 7') describes a positional distribution (X₁', X₂') of the model landmarks (1a', 3', 4', 5', 6', 7'), and the information concerning the spatial pattern of the positions of the patient landmarks (1a, 3, 4, 5, 6, 7) describes a positional distribution (X₁, X₂) of the patient landmarks (1a, 3, 4, 5, 6, 7).

5. The method according to any one of the preceding claims, wherein positional criteria are provided which describe criteria, which are dependent on the region of the body, for the position of the patient landmarks (1a, 3, 4, 5, 6, 7) with respect to each other and/or for the position of the model landmarks (3', 4', 5', 6', 7) with respect to each other.

6. The method according to any one of the preceding claims, wherein determining (S6) the body region includes determining an angle (α) between a straight line (a) through at least two particular patient landmarks (6, 7) and/or model landmarks (6', 7') and a perpendicular (b) with respect to a surface section of the part of the patient's body, wherein the position of one of the particular patient landmarks (6, 7) and/or model landmarks (6', 7') includes the surface section.

7. The method according to any one of the preceding claims, wherein the region of the body is determined to be a left-hand or right-hand side of the body.

8. The method according to any one of the preceding claims, wherein information concerning an anterior-posterior (A-P) direction (28) of the part of the patient's body is determined.

9. The method according to any one of the preceding claims, wherein the region of the body is determined to be an anterior or posterior side of the body.

10. The method according to any one of the preceding claims, wherein information concerning the position of a tibial axis (15) is determined.

11. The method according to any one of the preceding claims, wherein guidance information is outputted (S8) to a user on the basis of determining (S6) the region of the patient's body.

12. A program which, when it is loaded on a computer, causes the computer to perform the steps of the method according to any one of the preceding claims.

13. A navigation system designed to determine the region of the body in which an anatomical part of the body (2, 9) is situated, said navigation system including:
• a pointer (25) for determining anatomical point data which include information concerning a spatial pattern of positions of patient landmarks (1a, 3, 4, 5, 6, 7) of a part of a patient's body which corresponds to the anatomical part of the body (2, 9);
• a computer-readable storage medium (22) on which landmark data are stored which include information concerning a spatial pattern of positions of model landmarks (1a', 3', 4', 5', 6', 7') of a model of the anatomical part of the patient's body (2, 9);
• a surgical navigation system (17, 18, 20, 21, 22, 23) for comparing (S6) the anatomical point data with the landmark data,
wherein the surgical navigation system (17, 18, 20, 21, 22, 23) includes a data processing device (20, 21, 22) which is configured to perform the program according to claim 12.

14. The navigation system according to the preceding claim, which includes a computer-readable storage medium (22) on which the program according to claim 13 is stored.

15. The navigation system according to any one of the preceding two claims, which comprises a guidance information device (23, 24) for outputting (S8) guidance information which is based on the comparison between the landmark data and the anatomical point data.

## Revendications

1. Procédé pour déterminer la région du corps dans laquelle se trouve une partie anatomique du corps (2, 9), comportant les étapes suivantes consistant à :
• fournir (S3) des données de points anatomiques incluant des informations concernant un modèle spatial de positions de points de repère de patient (1a, 3, 4, 5, 6, 7) d'une partie du corps d'un patient, qui correspondent à la partie anatomique du corps (2, 9), les points de repère concernés du patient se situant sur la partie concernée du corps du patient,
• fournir (S4, S7) des données de points de repère incluant des informations concernant un modèle spatial de positions de points de repère de modèle (1a', 3', 4', 5', 6', 7') défini dans un modèle de la partie anatomique du corps (2, 9) du patient,
dans lequel, en supposant
• que les points de repère de patient (1a, 3, 4, 5, 6, 7) représentent les mêmes points de repère que les points de repère de modèle (1a', 3' , 4' , 5', 6', 7' ) et peuvent être mutuellement affectés de manière univoque,
sur la base des données de points de repère et des données de points anatomiques, on détermine la région du corps dans laquelle se trouve la partie du corps du patient,
**caractérisé en ce que** les points de repère de patient (1a, 3, 4, 5, 6, 7) incluent un point de repère artificiel (1a) sur la partie du corps du patient et des points de repère anatomiques (3, 4, 5, 6, 7) sur la partie du corps du patient, et les points de repère de modèle (1a', 3', 4', 5', 6', 7') incluent un point de repère artificiel (1a') dans le modèle et des points de repère anatomiques (3', 4', 5', 6', 7') dans le modèle.

2. Procédé selon la revendication précédente, dans lequel la position des points de repère artificiels correspond à la position d'un point de référence (1a), dans lequel la position du point de référence (1a) correspond en particulier à la position d'un dispositif de marqueur de partie du corps (1).

3. Procédé selon l'une quelconque des revendications précédentes, dans lequel les données de points anatomiques sont fournies (S3) de manière à ce qu'aucune affectation manuelle des positions des points de repère de patient (1a, 3, 4, 5, 6, 7) n'ait lieu par rapport à des positions de points de repère de modèle (1a', 3', 4', 5', 6', 7'), dans lequel les éléments du groupe de points de repère de patient (1a, 3, 4, 5, 6, 7) sont égaux aux éléments du groupe de points de repère de modèle (1a', 3', 4', 5', 6', 7').

4. Procédé selon l'une quelconque des revendications précédentes, dans lequel les informations concernant le modèle spatial des positions des points de repère de modèle (1a', 3', 4', 5', 6', 7') décrivent une distribution de positions (X₁', X₂') des points de repère de modèle (1a', 3', 4', 5', 6', 7'), et les informations concernant le modèle spatial des positions des points de repère de patient (1a, 3, 4, 5, 6, 7) décrivent une distribution de positions (X₁, X₂) des points de repère de patient (1a, 3, 4, 5, 6, 7).

5. Procédé selon l'une quelconque des revendications précédentes, dans lequel sont déterminés des critères de position qui décrivent des critères dépendant de la région du corps, pour la position des points de repère de patient (1a, 3, 4, 5, 6, 7) les uns par rapport aux autres et/ou des points de repère de modèle (3' , 4' , 5' , 6', 7') les uns par rapport aux autres.

6. Procédé selon l'une quelconque des revendications précédentes, dans lequel la détermination (S6) de la région du corps inclut la détermination d'un angle (α) entre une droite (a) passant par au moins deux points de repère de patient (6, 7) et/ou points de repère de modèle (6', 7') déterminés, et d'une perpendiculaire (b) à une section de surface de la partie du corps du patient, dans lequel la position de l'un des points de repère de patient (6, 7) et/ou points de repère de modèle (6', 7') déterminés inclut la section de surface.

7. Procédé selon l'une quelconque des revendications précédentes, dans lequel la région du corps est un côté gauche ou droit du corps.

8. Procédé selon l'une quelconque des revendications précédentes, dans lequel des informations concernant la direction antérieure-postérieure (a-p) de la partie du corps du patient sont déterminées.

9. Procédé selon l'une quelconque des revendications précédentes, dans lequel la région du corps est un côté antérieur ou postérieur du corps.

10. Procédé selon l'une quelconque des revendications précédentes, dans lequel des informations concernant la position d'un axe de tibia (15) sont déterminées.

11. Procédé selon l'une quelconque des revendications précédentes, dans lequel des informations de guidage sont générées (S8) à l'attention d'un utilisateur, sur la base de la détermination (S6) de la région du corps du patient.

12. Programme qui, lorsqu'il est chargé sur un ordinateur, amène l'ordinateur à exécuter les étapes du procédé selon l'une quelconque des revendications précédentes.

13. Système de navigation adapté pour déterminer la région du corps dans laquelle se trouve une partie anatomique du corps (2, 9), lequel système de navigation incluant :
• un pointeur (25) pour déterminer des données de points anatomiques qui incluent des informations concernant un modèle spatial de positions de points de repère de patient (1a, 3, 4, 5, 6, 7) d'une partie du corps du patient qui correspond à la partie anatomique du corps (2, 9) .
• un support de mémorisation lisible sur ordinateur (22) sur lequel sont mémorisées des données de points de repère qui incluent des informations concernant un modèle spatial de positions de points de repère de modèle (1a', 3', 4', 5', 6', 7') d'un modèle de la partie anatomique du corps (2, 9) du patient,
• un système de navigation chirurgical (17, 18, 20, 21, 22, 23) pour comparer (S6) les données de points anatomiques aux données de points de repère,
dans lequel le système de navigation chirurgical (17, 18, 20, 21, 22, 23) inclut un dispositif de traitement de données (20, 21, 22) qui est configuré pour exécuter le programme selon la revendication 12.

14. Système de navigation selon la revendication précédente, incluant un support de mémorisation lisible sur ordinateur (22) sur lequel est mémorisé le programme selon la revendication 12.

15. système de navigation selon l'une quelconque des deux revendications précédentes, contenant un dispositif d'information de guidage (23, 24) pour générer (S8) des informations de guidage qui sont basées sur la comparaison entre les données de points de repère et les données de points anatomiques.
